# EUROPÄISCHE PATENTSCHRIFT

(11) **EP 2 097 046 B1**
(45) Veröffentlichungstag und Bekanntmachung des Hinweises auf die Patenterteilung: **24.04.2013**
(21) Anmeldenummer: 07856255.0
(22) Anmeldetag: 24.11.2007
(51) Int. Cl.: A61C 1/08, A61C 13/00, A61F 2/28, A61F 2/30

(54) **VERFAHREN ZUR HERSTELLUNG EINES IMPLANTATS**
METHOD FOR PRODUCING AN IMPLANT
PROCÉDÉ DE FABRICATION D'UN IMPLANT

(30) Priorität: 30.11.2006 DE 102006056945
(43) Veröffentlichungstag der Anmeldung: 09.09.2009
(73) Patentinhaber: SCHLEE, Markus, 91301 Forcheim (DE)
(72) Erfinder: SCHLEE, Markus, 91301 Forcheim (DE)
(74) Vertreter: Ehnis, Tobias
(86) Internationale Anmeldenummer: PCT/EP2007/010225
(87) Internationale Veröffentlichungsnummer: WO 2008/064840

(56) Entgegenhaltungen:
- EP-A- 0 097 001
- EP-A- 0 574 098
- WO-A-01/77988
- WO-A-95/15131
- WO-A-98/12995
- WO-A-03/030787
- WO-A1-01/08714
- DE-A1- 19 724 724
- DE-A1- 19 922 279
- US-A- 6 112 109

## Beschreibung

Die Erfindung betrifft ein Verfahren zur Herstellung eines Implantats zum Ersatz eines fehlenden Teils eines Knochens, insbesondere eines Kieferknochens, eines Menschen sowie das mittels dieses Verfahrens hergestellte Implantat.

Aus dem Stand der Technik ist es bekannt, einem Menschen Knochenmaterial zu entnehmen und es zu verwenden, um damit an einer anderen Stelle des Menschen einen fehlenden Teil eines Knochens aufzufüllen. Nachteilig ist dabei, dass zwei Operationen durchgeführt werden müssen und sich dadurch das Komplikationsrisiko erhöht.

Die DE 197 24 724 A1 offenbart ein Verfahren zur Herstellung eines Knochenaugmentats. Dabei kann bei der Materialwahl für das Knochenaugmentat auf körpereigene Knochensubstanz zurückgegriffen werden. Als Materialien können auch heterologe Knochenersatzmaterialien, wie z. B. enteiweißter boviner Knochen, verwendet werden.

Aus der WO 01/08714 A1 ist eine Knochenmatrix als Vehikel zur Freisetzung von Nukleinsäure bekannt. Bei der Knochenmatrix kann es sich um ein Knochentransplantat handeln, bei dem im Wesentlichen alle nicht-kollagene Proteine oder Nicht-Strukturkollagenproteine entfernt worden sind und welches native Kollagen-Matezialien enthält. Dazu kann zunächst ein gereinigtes Knochentransplantat bereitgestellt und mit einer Entfettungslösung in Kontakt gebracht werden, um ein gereinigtes, entfettetes Knochentransplantat bereitzustellen. Dieses kann dann mit einem chaotropen Agens in Kontakt gebracht werden, um nicht-kollagene Proteine oder Nicht-Strukturkollagenproteine zu entfernen.

Aufgabe der vorliegenden Erfindung ist es, ein Verfahren zur Herstellung eines Implantats und ein Implantat bereitzustellen, welches die Nachteile nach dem Stand der Technik vermeidet. Insbesondere soll ein Verfahren zur Herstellung eines Implantats zum Ersatz eines fehlenden Teils eines Knochens eines Menschen angegeben werden, welches keine Operation zur Entnahme von Knochenmaterial zur Herstellung des Implantats erfordert. Unter einem Implantat wird hier generell ein Teil verstanden, welches in den Körper eines Menschen eingesetzt wird. Das Teil kann dabei sowohl aus einem künstlichen als auch einem natürlichen Material bestehen. Sofern das Material aus dem Menschen, in dessen Körper das Teil eingesetzt wird, oder aus einem anderen Organismus stammt und das Material kein totes Material ist, handelt es sich bei dem Implantat um ein Transplantat.

Die Aufgabe wird durch die Merkmale der Ansprüche 1 und 21 gelöst. Zweckmäßige Ausgestaltungen der Erfindung ergeben sich aus den Merkmalen der Ansprüche 2 bis 20.

Erfindungsgemäß ist ein Verfahren zur Herstellung eines Implantats zum Ersatz eines fehlenden Teils eines Knochens eines Menschen mit folgenden Schritten vorgesehen:
a) Computergestützte Berechnung erster Daten, welche die dreidimensionale Form des vorhandenen Knochens spezifizieren,
b) Übermittlung der ersten Daten an einen Computer und Berechnen zweiter Daten mittels dieses Computers, wobei die zweiten Daten eine dreidimensionale Form für das Implantat spezifizieren, welche zumindest auf einer ersten Seite ein Gegenstück zu zumindest einem Teil der Form des vorhandenen Knochens ist,
c) Übermitteln der zweiten Daten an eine computerunterstützte Fertigungsmaschine (CAM),
d) Erzeugen des Implantats mittels der computerunterstützten Fertigungsmaschine aus einer Matrix und
e) Sterilisieren des Implantats.

Die computergestützte Berechnung erster Daten, welche die dreidimensionale Form des vorhandenen Knochens spezifizieren, erfolgt auf der Grundlage von an einem Patienten ermittelten Messdaten. An dem zweiten Computer erfolgt die Gestaltung der dreidimensionalen Form des Implantats, wobei der Computer die zweiten Daten berechnet, welche diese dreidimensionale Form spezifizieren. Die Vorgaben für die Gestaltung können dabei von einem Nutzer eingegeben oder von dem Computer selbst berechnet werden.

Ein wesentlicher Vorteil des erfindungsgemäßen Verfahrens besteht darin, dass eine zusätzliche Operation zur Gewinnung des Knochenmaterials, beispielsweise aus dem Beckenkamm des Patienten, der das Implantat erhalten soll, entfällt. Dadurch ist kein Krankenhausaufenthalt des Patienten erforderlich und der Patient wird weniger belastet. Dadurch werden Kosten im Gesundheitswesen eingespart. Der volkswirtschaftliche Schaden wird ebenfalls verringert, da der Patient weniger lange arbeitsunfähig ist. Ein weiterer Vorteil des erfindungsgemäßen Verfahrens besteht darin, dass das Implantat mit hoher Präzision hergestellt werden kann. Dadurch passt es genau auf den vorhandenen Rest des Knochens, unabhängig davon, welche Form dieser aufweist. Es ist daher nicht notwendig, den vorhandenen Knochen zu bearbeiten. Eine derartige Präzision bei der Fertigung ist bei der Entnahme von Knochenmaterial und dessen sofortiger Bearbeitung im Operationssaal mit Handwerkzeugen nicht möglich. Weiterhin verringert sich dadurch, dass weder das Implantat noch der Knochen während der Operation bearbeitet werden muss, die Operationszeit deutlich. Dadurch wird das Komplikationsrisiko, beispielsweise durch Infektionen oder Absterben von Gewebe, deutlich verringert. Weiterhin erfolgt nach der Operation eine bessere und schnellere Heilung, weil der Eingriff insgesamt weniger invasiv ist als bei herkömmlichen Verfahren, bei denen eine Bearbeitung des vorhandenen Knochens erforderlich ist.

Das sterilisierte Implantat kann bei einer Operation an dem vorhandenen Knochen, beispielsweise durch Verschrauben, so angebracht werden, dass es das fehlende Teil des Knochens ersetzt. Die hohe Passgenauigkeit des Materials ermöglicht ein zügiges und problemloses Einheilen des Implantats sowie bei Belastung des Knochens und des Implantats eine Kräfteverteilung, welche der Kräfteverteilung beim ursprünglich vorhandenen Knochen entspricht. Beim Einheilen wird das Implantat von Knochenzellen besiedelt. Die Knochenzellen können neues Knochenmaterial bilden. Außerdem können Blutgefäße in das Implantat einwandern. Durch die Bildung des neuen Knochenmaterials wird das Implantat in den vorhandenen Knochen integriert. Je nach Matrix, aus welcher das Implantat besteht, kann alternativ oder zusätzlich auch eine Resorption der Matrix und ein Ersatz des resorbierten Materials durch neu gebildetes Knochenmaterial erfolgen. Das kann insbesondere bei einer Matrix aus Knochengewebe erfolgen.

Durch die Übermittlung erster Daten an einen Computer und der zweiten Daten an eine computerunterstützte Fertigungsmaschine können die Berechnung der ersten Daten, die Berechnung der zweiten Daten und die Fertigung des Implantats sowie das Einsetzen des Implantats räumlich und zeitlich getrennt voneinander erfolgen, so dass für diese Prozesse jeweils technisch bzw. beim Einsetzen des Implantats auch medizinisch optimale Bedingungen geschaffen werden können.

Darüber hinaus ermöglicht die räumliche und zeitliche Trennung, dass jeder Schritt von einer dafür jeweils spezialisierten Person an einem beliebigen Ort durchgeführt wird.

Das erfingdungsgemäß hergestellte Implantat ist geeignet, um beispielsweise einen nach einer Zahnextraktion zurückgebildeten Kieferknochen wieder so aufzubauen, dass nach dem Einheilen des Implantats ein Zahnimplantat in das Implantat bzw. das neu gebildete Knochenmaterial eingesetzt werden kann. Das Implantat ist weiterhin dazu geeignet, beispielsweise durch eine Tumorerkrankung oder einen Unfall zerstörtes Knochengewebe zu ersetzen.

Die ersten Daten können auf Grundlage weiterer Daten berechnet werden, welche von dem Knochen durch Vermessen mittels eines Computertomografen ermittelt werden. Auf der Grundlage derartiger weiterer Daten können erste Daten ermittelt werden, welche ein hochpräzises Abbild des vorhandenen Knochens spezifizieren. Vorzugsweise werden die zweiten Daten mittels computerunterstützter Konstruktion (CAD = Computer Aided Design) moduliert und berechnet.

Die Matrix, aus welcher das Implantat mittels der computerunterstützten Fertigungsmaschine herausgearbeitet wird, besteht aus menschlichem Knochengewebe. Das menschliche Knochengewebe für die Herstellung des Implantats fällt üblicherweise beim Ersatz eines Hüftgelenks durch ein künstliches Hüftgelenk als Abfallprodukt an. Bevorzugt handelt es sich bei dem Knochengewebe um Knochengewebe aus dem Oberschenkel und insbesondere aus dem oberschenkelkopf. Dieses Knochengewebe weist eine hohe Dichte auf und ist dadurch einerseits besonders stabil und bietet dadurch andererseits einwandernden Knochenzellen eine besonders große Oberfläche, um diese zu besiedeln. Das menschliche Knochengewebe stammt vorzugsweise von einem weiteren Menschen. Menschliches Knochengewebe hat für das erfindungsgemäße Verfahren den Vorteil, dass dieses so aufbereitet werden kann, dass das darin enthaltene Kollagen erhalten bleibt. Menschliches Knochengewebe hat gegenüber tierischem Knochengewebe den Vorteil, dass das darin enthaltene Kollagen humanes Kollagen ist. Im Gegensatz zu tierischem Kollagen löst dieses Kollagen nach der Implantierung keine oder nur eine geringe Immunreaktion oder Entzündungsreaktion aus. An das Kollagen können vor dem Implantieren des Implantats Wachstumsfaktoren gebunden werden, indem das Implantat in einer wässrigen Lösung dieser Wachstumsfaktoren inkubiert wird. Nach dem Implantieren des Implantats können an das Kollagen körpereigene Wachstumsfaktoren binden. Weiterhin begünstigt das Kollagen die Besiedelung des Implantats mit auch Osteoblasten genannten knochenbildenden Zellen (Knochenzellen). Diese Zellen adherieren an Kollagen und bilden die unverkalkte Interzellularsubstanz (Osteoid) des Knochens. Die Wachstunsfaktoren können die Besiedelung des Implantats mit Osteoblasten und das Einheilen des Implantats beschleunigen.

Das Knochengewebe ist denaturiertes, dehydratisiertes, entfettetes und Kollagen enthaltendes Knochengewebe. Das ist besonders vorteilhaft für die Einheilung des Implantats. Vorzugsweise ist die Form des Implantats weiterhin dadurch spezifiziert, dass sie auf einer zweiten Seite zumindest teilweise die Form des fehlenden Teils des Knochens nachbildet. Bei dem Knochen kann es sich um einen Schädelknochen, einen, insbesondere atrophischen, Kieferknochen, einen Knochen einer Extremität oder einen durch einen Unfall oder einen Tumor beschädigten Knochen handeln.

Besonders vorteilhaft ist es, wenn die Übermittlung der ersten und/oder der zweiten Daten über das Internet erfolgt, weil dadurch die Errechnung der zweiten Daten und die Fertigung des Implantats überall auf der Welt erfolgen kann. Weiterhin wird dadurch ein bereits vorhandenes Datennetz zur Übertragung der Daten verwendet, so dass dazu kein neues Datennetz bereitgestellt werden muss.

Das Knochengewebe wird dadurch bereitgestellt, dass Gewebe eines menschlichen weiteren Knochens, insbesondere eines Oberschenkelknochens oder Schienbeinknochens, zur Entfettung mit Aceton gespült, dann zur osmotischen Behandlung in einer hypotonen Kochsalzlösung inkubiert, anschließend zur oxidativen Behandlung in einer Wasserstoffperoxidlösung inkubiert und danach zum Wasserentzug in einem flüssigen Trocknungsmittel, wie z. B. Aceton, inkubiert und zuletzt getrocknet wird. Durch die osmotische Behandlung werden in dem Knochengewebe enthaltene Zellen, zumindest weitgehend, zerstört. Durch die Behandlung mit Aceton werden nicht nur Fette entfernt, sonders es wird auch die Antigenität von in dem Knochengewebe vorhandenen Proteinen zumindest stark herabgesetzt. Dadurch erfolgt, wenn überhaupt, nur eine schwache Immunreaktion nach dem Implantieren des Implantats, so dass dessen Verträglichkeit durch die Acetonbehandlung wesentlich erhöht wird. Ein derartig aufbereitetes Knochengewebe enthält noch das im Knochen ursprünglich vorhandene Kollagen und die mineralische Knochenstruktur, ist jedoch frei von potentiellen Krankheitserregern. Das Kollagen bleibt bei dem Verfahren, zumindest weitgehend, in seiner nativen dreidimensionalen Struktur erhalten. Dies ist besonders wichtig für eine gute Besiedlung des Implantats mit Knochenzellen und begünstigt die besonders vorteilhafte Resorption der das Implantat bildenden Matrix und dessen Ersetzung durch neu gebildetes Knochenmaterial. Bei der im Stand der Technik üblichen thermischen Behandlung von Knochenmaterial bleibt das Kollagen dagegen nicht oder zumindest nicht in seiner nativen Struktur erhalten.

Bevorzugt wird das Implantat durch die computergestützte Fertigungsmaschine aus dem Knochengewebe ausgefräst.

Das Sterilisieren des Implantats bei Schritt lit. e) kann mittels radioaktiver Strahlung, insbesondere Gammastrahlung, erfolgen. Dies hat den Vorteil, dass der Energieaufwand dafür gering ist, weil dazu keine Wärme aufgewandt werden muss. Durch das Vermeiden einer Erwärmung kann weiterhin vermieden werden, dass das Implantat durch die Wärme beschädigt wird, z. B. indem es sich verformt oder die biologische Wirksamkeit des darin enthaltenen Kollagens zerstört wird. Vor oder nach Durchführung der Sterilisierung des Implantats kann das Implantat in eine keimdichte Umhüllung, vorzugsweise einen Blister, insbesondere durch Einschweißen, verpackt werden. In der Umhüllung kann das Implantat transportiert und bis zur Operation aufbewahrt werden.

Bei einem Implantat, bei welchem zuvor enthaltenes Wasser entzogen worden ist, beispielsweise durch Inkubation mit Aceton, ist es vorteilhaft, wenn das Implantat unmittelbar vor dessen Implantieren durch Inkubation in einer wässrigen Lösung, vorzugsweise für etwa 30 Minuten, rehydriert wird. Dadurch wird das Implantats vom Körper besser angenommen und die Verträglichkeit und das spätere Einheilen werden verbessert.

Die wässrige Lösung kann mindestens einen Faktor enthalten. Der Faktor kann an im Implantat enthaltendes Kollagen binden und später das Besiedeln des Implantats mit Knochenzellen sowie deren Aktivität begünstigen und damit das Einheilen des Implantats verbessern und beschleunigen. Die wässrige Lösung kann auch eine Suspension von Zellen enthalten. Bei den Zellen kann es sich um Knochenzellen oder Stammzellen handeln. Dadurch kann das Einheilen weiter beschleunigt werden, weil der Einheilprozess bereits beginnen kann bevor das Implantat von körpereigenen Zellen besiedelt ist.

Besonders vorteilhaft ist es, wenn das Implantat vor dem Verpacken in einer mindestens einen Faktor enthaltenden wässrigen Lösung inkubiert und dann in einer weiteren oder derselben wässrigen Lösung in der Umhüllung verpackt wird. Dadurch kann das Implantat sofort nach der Entnahme aus der Umhüllung implantiert werden. Eine Rehydrierung mit einer den Faktor enthaltenden wässrigen Lösung unmittelbar vor dem Implantieren entfällt. Vorzugsweise wird dem Implantat beim Verpacken eine mindestens einen Faktor enthaltende wässrige Lösung mit mindestens einem Wachstumsfaktor zugesetzt. Die wässrige Lösung ist vorzugsweise eine wässrige Kochsalzlösung. Der Faktor kann ein Zytokin, ein Wachstumsfaktor, ein Bone Morphogenic Protein (BMP) oder ein die Bildung von Blutgefäßen begünstigender Faktor, insbesondere ein Vascular Endothelial Growth Factor (VEGF), vorzugsweise ein VEGF-A, sein. Besonders vorteilhaft ist es, wenn der Faktor ein Kollagen bindender Faktor ist, weil er dann besonders gut an einem Kollagen enthaltenden Implantat haften kann. Dieser kann dadurch im Implantat langanhaltend wirkten weil er von dort nicht ohne Weiteres von Körperflüssigkeiten weggeschwemmt werden kann. Er kann so eine gute und schnelle Einheilung des Implantats fördern.

Die Erfindung betrifft weiterhin ein nach dem erfindungsgemäßen Verfahren hergestelltes Implantat.

Die Erfindung wird nachfolgend anhand der Figuren näher erläutert. Es zeigen:
- Fig. 1: einen Ablaufplan, welcher das erfindungsgemäße Verfahren zur Herstellung des Implantats umfasst,
- Fig. 2: verschiedene computertomografische Aufnahmen eines Unterkiefers und eine dreidimensionale Darstellungen des Kieferknochens mit aufgesetzten Implantaten,
- Fig. 3: verschiedene dreidimensionale Darstellungen des atrophierten Unterkieferknochens, zum Teil mit aufgesetztem Implantat,
- Fig. 4a, b: verschieden geschnittene Teile eines menschlichen Oberschenkelknochens und
- Fig. 5a: das fertiggestellte Implantat und
- Fig. 5b: das Implantat während der Fertigung.

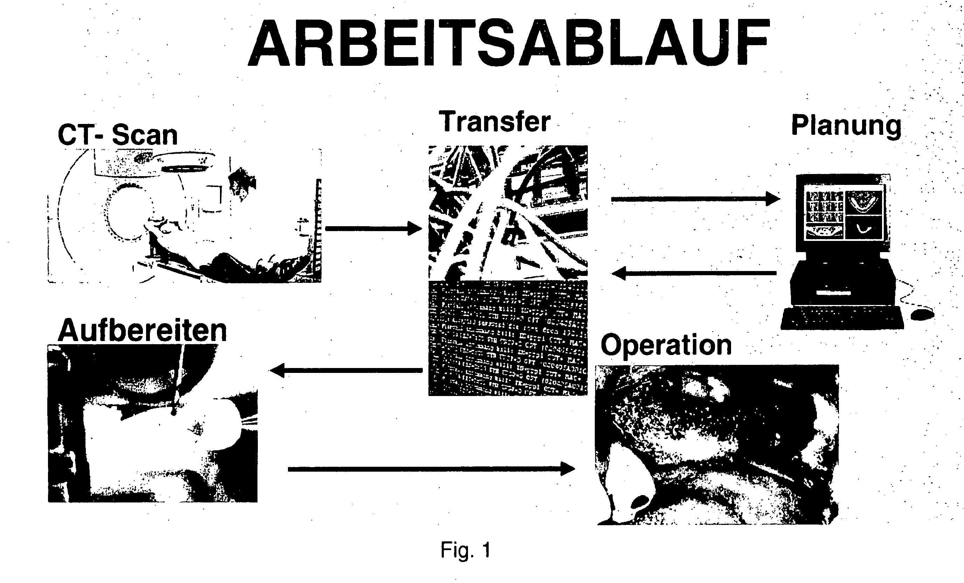

In der ersten Abbildung des Ablaufplans gemäß Fig. 1 ist das Vermessen des Knochens mittels eines Computertomografen dargestellt. Aus den dabei ermittelten weiteren Daten werden computergestützt erste Daten berechnet, welche die dreidimensionale Form des vorhandenen Knochens spezifizieren. Diese ersten Daten werden, vorzugsweise über das Internet, an einen Computer übermittelt, wo die Berechnung zweiter Daten, die eine dreidimensionale Form für das Implantat spezifizieren, erfolgt. Die dreidimensionale Form ist zumindest auf einer Seite ein Gegenstück zu zumindest einem Teil der Form des vorhandenen Knochens. Die berechneten zweiten Daten werden, wiederum bevorzugt über das Internet, an eine computergestützte Fertigungsmaschine übermittelt, mittels welcher das Implantat aus einer Matrix aufbereitet wird. Nach einer Sterilisierung des Implantats kann dieses zur Implantierung, beispielsweise wie hier dargestellt in einen Kiefer, an eine entsprechende Einrichtung, beispielsweise eine chirurgische Abteilung eines Krankenhauses, verschickt werden.

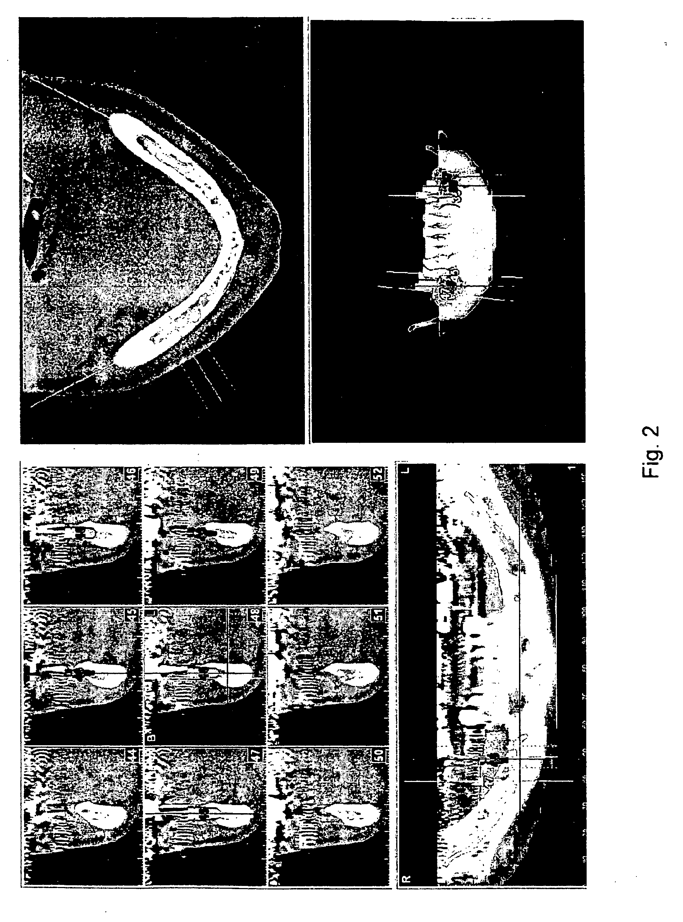

Fig. 2 zeigt in den links oben angeordneten Bildern 44 bis 52 verschiedener Schnittebenen eines Unterkiefers, in welchem ein Teil des Kieferknochens durch Atrophie abgebaut worden ist. In der unteren linken Darstellung ist ein aus Schnittbildern zusammengesetztes Bild des Unterkieferknochens in einer Ansicht von vorne und in der oberen rechten Darstellung in der Aufsicht zu sehen. Unten rechts ist eine aus den Daten der Schnittbilder errechnete dreidimensionale Darstellung des Kieferknochens einschließlich auf der linken und der rechten Hälfte des Kieferknochens aufgesetzter Implantate zu sehen. Die dreidimensionale Form der Implantate ist auf der Grundlage der ersten Daten, welche mittels der Computertomografie ermittelt worden sind, berechnet worden.

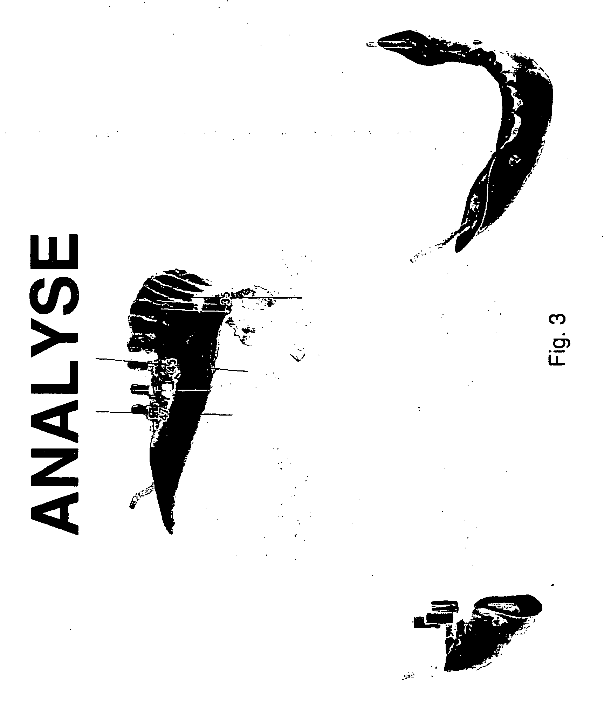

In Fig. 3 sind verschiedene dreidimensionale Darstellungen des atrophierten Unterkieferknochens zu sehen, welche der Analyse der Situation am Unterkieferknochen und der Planung und Berechnung der Form des Implantats in Form der zweiten Daten dienen. Bei der oberen Darstellung der Fig. 3 ist bereits das aufgesetzte Implantat zusammen mit dem Kieferknochen und später in das Implantat einzusetzenden Träger für Zahnprothesen dargestellt.

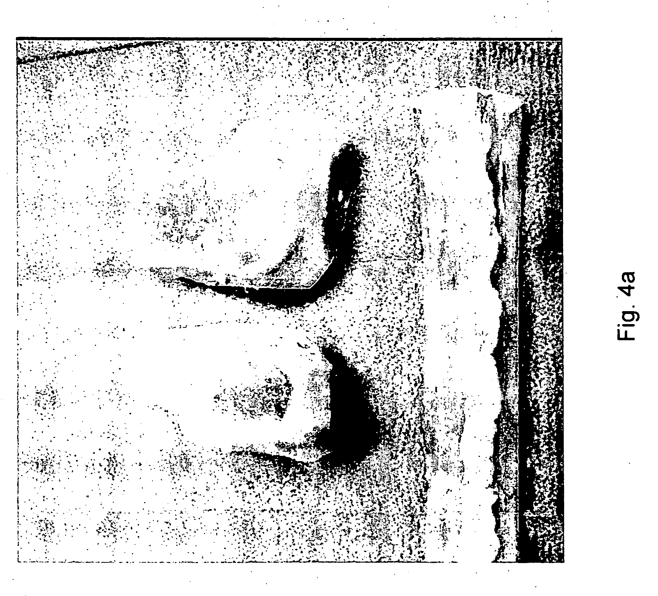

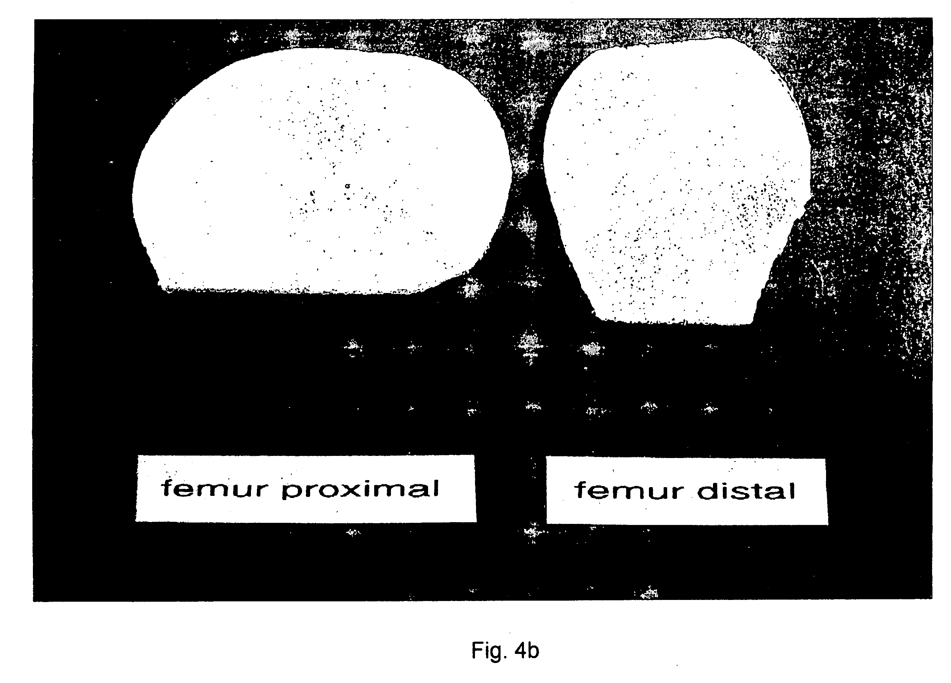

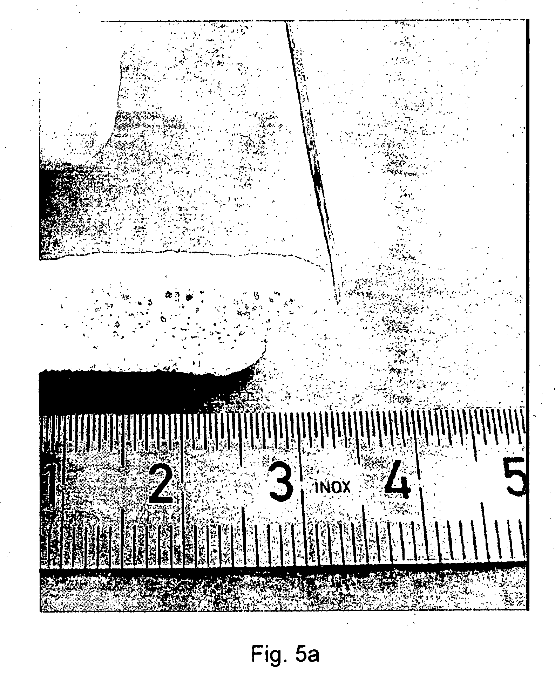

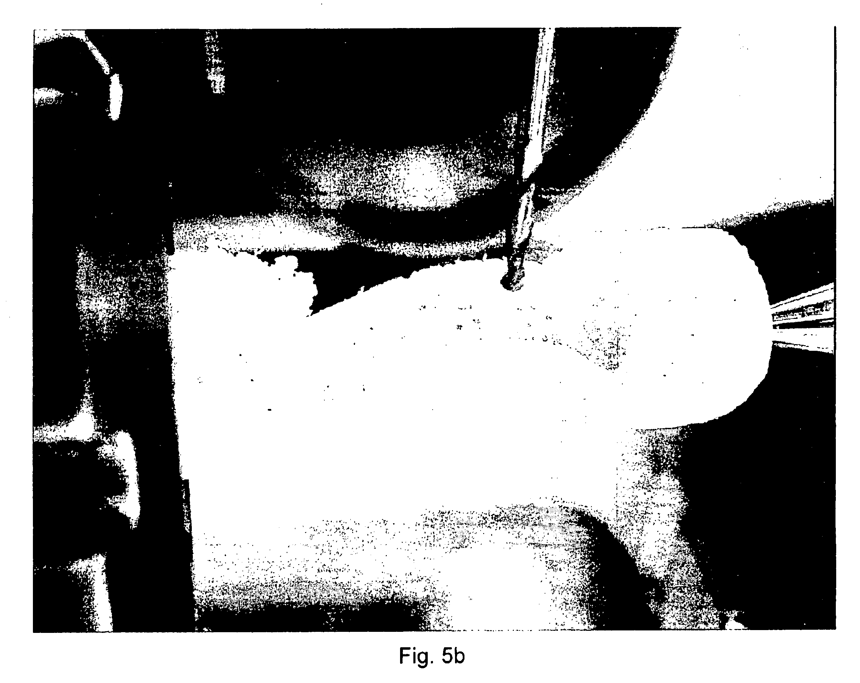

Fig. 4a zeigt oben zwei verschieden geschnittene menschliche Oberschenkelköpfe und das darin enthaltene Knochengewebe und unten aus einem Oberschenkelknochen herausgearbeitetes Knochengewebe. Aus dem Knochengewebe der Oberschenkelköpfe kann das Implantat hergestellt werden. Fig. 4b zeigt je einen proximalen und einen distalen Schnitt durch einen humanen Oberschenkelkopf. Daraus ist gut die hohe Dichte des im Oberschenkelkopf enthaltenen Knochengewebes zu ersehen. Fig. 5a zeigt das fertiggestellte Implantat und Fig. 5b das Implantat während der Fertigung in der computerunterstützten Fertigungsmaschine.

## Patentansprüche

1. Verfahren zur Herstellung eines Implantats zum Ersatz eines fehlenden Teils eines Knochens eines Menschen mit folgenden Schritten:
a) Computergestützte Berechnung erster Daten, welche die dreidimensionale Form des vorhandenen Knochens spezifizieren,
b) Übermittlung der ersten Daten an einen Computer und Berechnen zweiter Daten mittels dieses Computers, wobei die zweiten Daten eine dreidimensionale Form für das Implantat spezifizieren, welche zumindest auf einer ersten Seite ein Gegenstück zu zumindest einem Teil der Form des vorhandenen Knochens ist,
c) Übermitteln der zweiten Daten an eine computerunterstützte Fertigungsmaschine (CAM),
d) Erzeugen des Implantats mittels der computerunterstützten Fertigungsmaschine aus einer Matrix, aus welcher das Implantat mittels der computerunterstützten Fertigungsmaschine herausgearbeitet wird und
e) Sterilisieren des Implantats,
wobei die Matrix aus menschlichem Knochengewebe besteht, wobei das Knochengewebe denaturiertes, dehydratisiertes, entfettetes und Kollagen enthaltendes Knochengewebe ist, wobei das Knochengewebe dadurch bereitgestellt wird, dass Gewebe eines menschlichen weiteren Knochens zur Entfettung mit Aceton gespült, dann zur osmotischen Behandlung in einer hypotonen Kochsalzlösung inkubiert, anschließend zur oxidativen Behandlung in einer Wasserstoffperoxydlösung inkubiert und danach zum Wasserentzug in einem flüssigen Trocknungsmittel inkubiert und zuletzt getrocknet wird.

2. Verfahren nach Anspruch 1, wobei die ersten Daten auf der Grundlage weiterer Daten berechnet werden, welche von dem Knochen durch Vermessen mittels eines Computertomografen ermittelt werden.

3. Verfahren nach einem der vorherigen Ansprüche, wobei die zweiten Daten mittels computerunterstützter Konstruktion (CAD) moduliert und berechnet werden.

4. Verfahren nach einem der vorherigen Ansprüche, wobei die Form für das Implantat weiterhin dadurch spezifiziert ist, dass sie auf einer zweiten Seite zumindest teilweise die Form des fehlenden Teils des Knochens nachbildet.

5. Verfahren nach einem der vorherigen Ansprüche, wobei die Übermittlung der ersten und/oder der zweiten Daten über das Internet erfolgt.

6. Verfahren nach einem der Ansprüche 1 bis 5, wobei der menschliche weitere Knochen ein Oberschenkelknochen oder Schienbeinknochen ist.

7. Verfahren nach einem der Ansprüche 1 bis 6, wobei das Implantat durch die computerunterstützte Fertigungsmaschine aus dem Knochengewebe ausgefräst wird.

8. Verfahren nach einem der vorherigen Ansprüche, wobei das Implantat vor oder nach Schritt lit. e) in eine keimdichte Umhüllung, vorzugsweise einen Blister, insbesondere durch Einschweißen, verpackt wird.

9. Verfahren nach einem der vorherigen Ansprüche, wobei das Implantat unmittelbar vor dessen Implantieren durch Inkubation in einer wässrigen Lösung, insbesondere in einer physiologischen Kochsalzlösung, vorzugsweise für 30 Minuten, rehydriert wird.

10. Verfahren nach Anspruch 9, wobei die wässrigen Lösung mindestens einen Faktor, insbesondere ein Zytokin, ein Wachstumsfaktor, ein Bone Morphogenic Protein (BMP) oder ein die Bildung von Blutgefäßen begünstigender Faktor, insbesondere ein Vascular Endothelial Growth Factor (VEGF), vorzugsweise ein VEGF-A, oder eine Suspension von Zellen, insbesondere Knochenzellen oder Stammzellen, enthält.

11. Verfahren nach Anspruch 8, wobei das Implantat vor dem Verpacken in einer mindestens einen Faktor enthaltenden wässrigen Lösung inkubiert und dann in einer weiteren oder derselben wässrigen Lösung in der Umhüllung verpackt wird.

12. Verfahren nach Anspruch 8, wobei dem Implantat beim Verpacken eine mindestens einen Faktor enthaltende wässrige Lösung mit mindestens einem Wachstumsfaktor zugesetzt wird.

13. Verfahren nach einem der Ansprüche 10 bis 12, wobei der Faktor ein Kollagen bindender Faktor ist.

14. Verfahren nach einem der vorherigen Ansprüche, wobei das flüssige Trocknungsmittel Aceton ist.

15. Implantat hergestellt nach einem Verfahren gemäß einem der vorhergehenden Ansprüche.

## Claims

1. Method for making an implant for the replacement of a missing part of a bone of a human being using the following steps:
a) Computer-supported calculation of first data which specify the three-dimensional form of the existing bone,
b) Transmission of the first data to a computer and calculation of second data via this computer, wherein the second data specify a three-dimensional form for the implant which is at least on a first side a counterpart to at least one part of the form of the existing bone,
c) Transmission of the second data to a computer-aided manufacturing machine (CAM),
d) Generation of the implant via the computer-aided manufacturing machine from a matrix from which the implant is worked out via the computer-aided manufacturing machine and
e) Sterilization of the implant,
wherein the matrix is made of human bone tissue, wherein the bone tissue is denaturized, dehydrated, degreased bone tissue that contains collagen, wherein the bone tissue is provided by degreasing tissue of an additional human bone by flushing with acetone, then incubating in a hypotonic sodium chloride solution for osmotic treatment, subsequently incubating in a hydrogen peroxide solution for oxidative treatment and afterwards incubating in a liquid drying agent for dehydration and then finally drying.

2. Method as defined in claim 1, wherein the first data are calculated based on additional data which are determined from the bone by measuring via a computer tomography scanner.

3. Method as defined in one of the preceding claims, wherein the second data are modulated and calculated via computer-aided design (CAD).

4. Method as defined in one of the preceding claims, wherein the form for the implant is further specified in that, on a second side, the form of the missing part of the bone is at least partially simulated.

5. Method as defined in one of the preceding claims, wherein the first and/or second data are transmitted via Internet.

6. Method as defined in one of the claims 1 to 5, wherein the further human bone is a thigh bone or a shinbone.

7. Method as defined in one of the claims 1 to 6, wherein the implant is machined out of the bone tissue by the computer-aided manufacturing machine.

8. Method as defined in one of the preceding claims, wherein before or after step lit. e) the implant is packed in a germ-proof wrap, preferably a blister wrap, in particular by shrink-wrapping.

9. Method as defined in one of the preceding claims, wherein immediately before the implant is implanted, it is rehydrated preferably for 30 minutes by incubation in a watery solution, in particular in a physiological sodium chloride solution.

10. Method as defined in claim 9, wherein the watery solution contains at least one factor, in particular a cytokine, a growth factor, a bone morphogenic protein (BMP) or a factor that encourages the creation of blood vessels, in particular a vascular endothelial growth factor (VEGF), preferably a VEGF-A, or a suspension of cells, in particular bone cells or stem cells.

11. Method as defined in claim 8, wherein, before being packaged, the implant is incubated in a watery solution containing at least one factor and then packaged in the wrap in a further or the same watery solution.

12. Method as defined in claim 8, wherein a watery solution containing at least one factor with at least one growth factor is added to the implant during packaging.

13. Method as defined in one of the claims 10 to 12, wherein the factor is a collagen-binding factor.

14. Method as defined in one of the preceding claims, wherein the liquid drying agent is acetone.

15. Implant made in accordance with a method as per one of the preceding claims.

## Revendications

1. Procédé de fabrication d'un implant pour le remplacement d'une partie manquante d'un os d'un homme, comprenant les étapes suivantes:
a) calcul assisté par ordinateur de premières données qui spécifient la forme tridimensionnelle de l'os présent,
b) transmission des premières données à un ordinateur et calcul de secondes données au moyen de cet ordinateur, dans lequel les secondes données spécifient une forme tridimensionnelle pour l'implant qui est au moins sur un premier côté un pendant à au moins une partie de la forme de l'os présent,
c) transmission des secondes données à une machine de fabrication assistée par ordinateur (FAO),
d) production de l'implant au moyen de la machine de fabrication assistée par ordinateur à partir d'une matrice à partir de laquelle l'implant est ébauché au moyen de la machine de fabrication assistée par ordinateur et
e) stérilisation de l'implant,
dans lequel la matrice se compose de tissu osseux humain, dans lequel le tissu osseux est du tissu osseux dénaturé, déshydraté, dégraissé et contenant du collagène, dans lequel le tissu osseux est préparé en ce que du tissu d'un autre os humain est rincé à l'acétone pour le dégraissage, puis incubé dans une solution de sel de cuisine hypotonique pour le traitement osmotique, ensuite incubé dans une solution de peroxyde d'hydrogène pour le traitement oxydatif et après incubé dans un agent de séchage liquide pour la déshydratation et enfin séché.

2. Procédé selon la revendication 1, dans lequel les premières données sont calculées sur la base d'autres données qui sont déterminées par l'os par mesure au moyen d'un tomographe informatique.

3. Procédé selon l'une quelconque des revendications précédentes, dans lequel les seconde données sont modulées et calculées au moyen d'une conception assistée par ordinateur (CAO).

4. Procédé selon l'une quelconque des revendications précédentes, dans lequel la forme pour l'implant est par ailleurs spécifié en ce qu'elle reproduit sur un second côté au moins partiellement la forme de la partie manquante de l'os.

5. Procédé selon l'une quelconque des revendications précédentes, dans lequel la transmission des premières et/ou des secondes données s'effectue par Internet.

6. Procédé selon l'une quelconque des revendications 1 à 5, dans lequel l'autre os humain est un os de fémur ou un os de tibia.

7. Procédé selon l'une quelconque des revendications 1 à 6, dans lequel l'implant est fraisé à partir du tissu osseux par la machine de fabrication assistée par ordinateur.

8. Procédé selon l'une quelconque des revendications précédentes, dans lequel l'implant est emballé dans une enveloppe étanche aux bactéries, de préférence un emballage cloque, notamment par soudure, avant ou après l'étape e).

9. Procédé selon l'une quelconque des revendications précédentes, dans lequel l'implant est réhydraté directement avant son implantation par incubation dans une solution aqueuse, notamment dans une solution de sel de cuisine physiologique, de préférence pendant 30 minutes.

10. Procédé selon la revendications9, dans lequel la solution aqueuse contient au moins un facteur, notamment une cytokine, un facteur de croissance, une protéine morphogénique osseuse (BMP) ou un facteur favorisant la formation de vaisseaux sanguins, notamment un facteur de croissance endothélial vasculaire (VEGF), de préférence un VEGF-A, ou une suspension de cellules, notamment de cellules osseuses ou de cellules souches.

11. Procédé selon la revendications8, dans lequel l'implant est incubé avant l'emballage dans une solution aqueuse contenant au moins un facteur, puis est emballé dans l'enveloppe dans une autre solution aqueuse ou la même.

12. Procédé selon la revendication 8, dans lequel une solution aqueuse contenant au moins un facteur avec au moins un facteur de croissante est ajoutée à l'implant lors de l'emballage.

13. Procédé selon l'une quelconque des revendications 10 à 12, dans lequel le facteur est un facteur se fixant au collagène.

14. Procédé selon l'une quelconque des revendications précédentes, dans lequel l'agent de séchage liquide est l'acétone.

15. Implant fabriqué selon un procédé selon l'une quelconque des revendications précédentes.
